# EUROPÄISCHE PATENTANMELDUNG

(11) **EP 3 536 227 A1**
(43) Veröffentlichungstag der Anmeldung: **11.09.2019**
(21) Anmeldenummer: 19161725.7
(22) Anmeldetag: 08.03.2019
(51) Int. Cl.: A61B 5/00, A61B 5/11, A63B 26/00, A63B 21/00, A63B 21/04, A63B 21/02

(54) **VORRICHTUNG UND VERFAHREN ZUM TRAINIEREN UND/ODER TESTEN DES MENSCHLICHEN KÖRPERS**

(30) Priorität: 09.03.2018 DE 102018105485; 15.05.2018 DE 102018111577
(71) Anmelder: Deubel, Gunther, 66583 Neunkirchen (DE); Deubel, Viviane, 66538 Neunkirchen (DE); Deubel, Marius, 66538 Neunkirchen (DE); Deubel, Robin, 66538 Neunkirchen (DE)
(72) Erfinder: Deubel, Gunther, 66583 Neunkirchen (DE); Deubel, Viviane, 66538 Neunkirchen (DE); Deubel, Marius, 66538 Neunkirchen (DE); Deubel, Robin, 66538 Neunkirchen (DE)
(74) Vertreter: Patentanwälte Bernhardt / Wolff Partnerschaft mbB

(57) **Zusammenfassung**

Die Erfindung betrifft eine Vorrichtung (1) zum Trainieren oder/und Testen des menschlichen Körpers, insbesondere zum Trainieren der Körperkoordination oder/und der Beinmuskulatur. Die Vorrichtung (1) ist gekennzeichnet durch einen Strahlgeber (3), der zur zielgerichteten Abgabe eines Strahls (4) vorgesehen ist, und eine Einrichtung (5) zur Befestigung des Strahlgebers (3) an einem Arm oder einem Bein des menschlichen Körpers, an welcher der Strahlgeber (3) angeordnet ist. In einer Ausgestaltung der Erfindung ist der Strahlgeber (3) dazu eingerichtet, einen Strahl (4) einer elektromagnetischen Welle abzugeben. Der Strahlgeber (3) ist vorzugsweise ein Laser, der einen Strahl Wellenlängenbereich des sichtbaren Lichts oder des Infrarotlichts abgibt. Die Befestigungseinrichtung (5) ist zweckmäßigerweise zur Anordnung an einem Gelenk des Arms oder des Beins, insbesondere des Knies des menschlichen Körpers eingerichtet und kann eine Knie-, eine Handgelenk- oder eine Ellbogenmanschette umfassen.

Die Erfindung betrifft ferner ein Verfahren zum Trainieren oder/und Testen des menschlichen Körpers.

## Beschreibung

Die Erfindung betrifft ein Verfahren und eine Vorrichtung zum Trainieren des menschlichen Körpers, insbesondere zum Trainieren und/oder Testen der Körperkoordination und/oder der Beinmuskulatur.

Es sind verschiedene Vorrichtungen und Verfahren, mit denen sich die Beinmuskulatur und die Körperkoordination trainieren lässt, bekannt. Ein hinlänglich bekanntes Fitnessgerät ist die Beinpresse, bei der mit den Beinen ein Gewicht gestemmt wird. Ein Verfahren, das ohne Fitnessgeräte durchgeführt werden kann, ist das sogenannte Beinachsentraining, bei dem zumindest ein Fuß mit gebeugtem Bein auf dem Boden aufgestellt wird, sodass der Fuß und das Bein ganz oder teilweise das Körpergewicht tragen und, ggf. unter Durchführung von Bewegungen mit dem restlichen Körper, auf die Stellung des Beins geachtet wird.

Der Erfindung liegt die Aufgabe zugrunde, eine Möglichkeit zu schaffen, die Durchführung des Trainingsverfahrens besser zu kontrollieren.

Bezüglich des Trainingsverfahrens wird die Aufgabe erfindungsgemäß dadurch gelöst, dass ein Strahlgeber an einem Arm oder einem Bein des menschlichen Körpers befestigt wird und ein von dem Strahlgeber ausgegebener Strahl, ggf. unter Bewegung des Arms oder des Beins, auf ein Zielobjekt gerichtet wird.

Hinsichtlich der Trainingsvorrichtung wird die Aufgabe durch einen Strahlgeber gelöst, der zur zielgerichteten Abgabe eines Strahls vorgesehen ist, und eine Einrichtung zur Befestigung des Strahlgebers an einem Arm oder einem Bein des menschlichen Körpers, an der der Strahlgeber angeordnet ist.

Bei Durchführung des erfindungsgemäßen Verfahrens erfordert die Ausrichtung des Strahls je nach Körperstellung und Dauer, über die die Körperstellung gehalten wird, Muskelkraft, Koordination und darüber hinaus eine gewisse Geschicklichkeit. Vorteilhaft kann der Trainierende während der Durchführung des Trainingsverfahrens direkt erkennen, ob der Strahl durch die Ausrichtung der Trainingsvorrichtung mit dem Arm oder dem Bein auf das Zielobjekt gerichtet ist und so überprüfen, ob der Arm oder das Bein in der für das Training vorgesehenen Position angeordnet ist.

Ein vergleichsweise einfache Übungsvariante des Trainingsverfahren ist, die Trainingsvorrichtung am Bein oder Arm zu befestigen, das Bein oder den Arm zu belasten, zu beugen und dabei das Bein oder den Arm in eine derartige Stellung zu bringen, dass der Strahlgeber den Strahl auf das Zielobjekt richtet, und diese Stellung möglichst lange zu halten. Vorteilhaft erkennt der Trainierende direkt, dass der Arm oder das Bein die vorgesehen Stellung verlässt, wenn der Strahl nicht mehr auf das Zielobjekt oder eine auf dem Zielobjekt vorgesehen Stelle gerichtet ist.

Es versteht sich, dass diese Übungsvariante im Stehen, im Sitzen oder im Liegen durchgeführt werden kann. Die Belastung des Beins im Stehen kann durch das Körpergewicht erfolgen. Im Liegen kann die Belastung z.B. dadurch aufgebracht werden, dass der Fuß in horizontaler Richtung gegen einen Gegenstand, beispielsweise einen Ball oder eine Wand gedrückt wird. Die Belastung des Arms könnte beispielsweise durch Halten eines Gewichts oder durch Abstützen des Körpers mittels des Arms erfolgen.

Vorstellbar wäre ferner, das Trainingsverfahren beim Stemmen einer Masse in einer Beinpresse zu verwenden. Dabei könnte ein Ziel sein, beim Stemmen der Masse mit dem Bein oder den Beinen, den Strahl auf einen schmalen, das Zielobjekt bildenden Bereich zu richten. Der Bereich weist dann vorzugsweise eine längliche, vorzugsweise Im Wesentlichen rechteckige, Form auf.

In einer Ausführungsform der Erfindung ist der Strahlgeber dazu eingerichtet, einen Energie- oder Materiestrom abzugeben. Vorzugsweise ist Strahl eine elektromagnetische Welle, insbesondere ein Lichtstrahl, vorzugsweise im Wellenlängenbereich des sichtbaren Lichts (λ = 380 bis 780 nm) oder der des Infrarotlichts (λ = 780 nm bis 1 mm).

Zweckmäßigerweise ist der Strahlgeber zur Abgabe eines Strahls eines Durchmessers < 3 mm, vorzugsweise < 2 mm, vorgesehen. Der Strahlgeber könnte beispielsweise ein Laser sein. Vorstellbar wäre ferner, dass der Strahlgeber zur Abgabe von Funkwellen vorgesehen ist.

In einer Ausgestaltung der Erfindung ist die Befestigungseinrichtung zur Anordnung an einem Gelenk des Arms oder des Beins, vorzugsweise des Handgelenks oder des Knies, eingerichtet. Die Befestigungseinrichtung weist zweckmäßigerweise eine Formung auf, die an die Form des Arms oder des Beins, an der sie zu befestigen ist, angepasst ist. Sie kann z.B. eine Auswölbung zur Aufnahme der Kniescheibe aufweisen.

Die Befestigungseinrichtung kann beispielsweise eine Knie-, eine Handgelenk- oder eine Ellbogenmanschette, eine Knie-, eine Handgelenk- oder eine Ellbogenorthese oder einen Knie-, eine Handgelenk- oder eine Ellbogenprotektor umfassen.

Die Befestigungseinrichtung könnte ferner durch eine Binde, die zur Befestigung am Arm oder am Bein vorgesehen ist, und eine Einrichtung zur Befestigung des Strahlgebers an der Binde umfassen. Die Einrichtung zur Befestigung des Strahlgebers an der Binde könnte beispielsweise durch einen Klettverschluss, durch eine Klammerhalterung oder dergleichen gebildet sein.

Zweckmäßigerweise ist die Trainingsvorrichtung zur Anordnung am Arm oder Bein vorgesehen derart, dass der Strahl vom menschlichen Körper, vorzugsweise in ventraler Richtung, wegstrahlt. Vorteilhaft kann das Zielobjekt beim Training ventral vor dem Körper angeordnet werden, damit es gut sichtbar ist, und beim Training oder Testen gut beobachtet werden kann.

In einer bevorzugten Ausführungsform der Erfindung weist der Strahlgeber eine Einrichtung zur Verstellung seiner Ausrichtung im Verhältnis zu der Befestigungseinrichtung auf, um die Richtung, in der der Strahlgeber den Strahl abgibt, einzustellen. Vorteilhaft lässt sich der Strahlgeber an der Befestigungseinrichtung in verschiedenen Positionen ausrichten, z.B. damit der Strahl bei einer bestimmten Körperhaltung auf das Zielobjekt trifft. Dadurch wird es möglich, die Vorrichtung an unterschiedliche Körpergrößen und/oder -formen oder/und verschiedene Körperstellungen anzupassen oder/und an unterschiedlichen Stellen am Arm oder am Bein zu befestigen und dabei den Strahl so auszurichten, dass die Stelle, an der er auftrifft, gut sichtbar ist.

Vorzugsweise ist die Verstelleinrichtung so gebildet bzw. wird bei Durchführung des Verfahrens so eingestellt, dass sich der Strahlgeber anordnen lässt derart, dass der Strahl im Wesentlichen senkrecht vom genannten Gelenk des Arms oder des Beins wegstrahlt.

Zweckmäßigerweise umfasst die Verstelleinrichtung ein Gelenk, vorzugsweise ein Drehgelenk oder ein Kugelgelenk.

In einer Ausgestaltung der Erfindung ist der Strahlgeber an der Befestigungseinrichtung an einer Stelle befestigt, die zur Anordnung an oder neben der Kniescheibe des Knies, insbesondere auf der Kniescheibe, vorgesehen ist.

In einer besonders bevorzugten Ausgestaltung der Erfindung umfasst die Vorrichtung ein Zielobjekt, auf das der Strahl zu richten ist. Zweckmäßigerweise ist das Zielobjekt in einem Abstand > 1 m, vorzugsweis > 2 m, von dem Strahlgeber angeordnet. Während es vorstellbar wäre, das Zielobjekt vorzusehen derart, dass der Strahl beim Auftreffen darauf sichtbar ist, weist es in einer Ausführungsform der Erfindung eine Einrichtung zum Detektieren des Auftreffens des Strahls auf, wobei die Detektiereinrichtung vorzugsweise einen Sensor umfasst, der zum Detektieren des Auftreffens des Strahls eingerichtet ist.

In einer weiteren Ausgestaltung der Erfindung umfasst die Vorrichtung eine Meldeeinrichtung, die dazu eingerichtet ist, zu melden, wenn der Strahl auf das Zielobjekt auftrifft. Die Meldeeinrichtung ist zweckmäßigerweise zur Abgabe eines optischen und/oder eines akustischen Signals vorgesehen und umfasst dazu vorzugsweise einen Lautsprecher oder einen Bildschirm. Diese Variante ist insbesondere interessant, wenn der Strahlgeber Licht im nicht sichtbaren Bereich abgibt. Auf dem Bildschirm wird dann durch die Anzeige der Auftrittsstelle eine optisch erkennbare Rückmeldung gegeben.

In einer Ausführungsform der Erfindung ist das Zielobjekt durch einen Bildschirm, insbesondere einen Touchscreen, gebildet. Das Zielobjekt ist bevorzugt vorgesehen derart, dass auf dem Bildschirm die Stelle, an der der Strahl auf das Zielobjekt auftrifft, angezeigt wird.

In einer Ausgestaltung der Erfindung umfasst die Vorrichtung einen Computer, der mit dem Zielobjekt, der Detektiereinrichtung und/oder der Meldeeinrichtung verbunden ist.

Zweckmäßigerweise ist auf dem Computer eine Software gespeichert, die anhand der mittels der Detektiereinrichtung ermittelten Informationen die Meldeeinrichtung zur Abgabe des optischen und des akustischen Signals veranlassen kann.

In einer Ausgestaltung der Erfindung ist die Vorrichtung, insbesondere mittels des Computers bzw. der Software, dazu vorgesehen, eine Dauer, innerhalb derer der Strahl auf das Zielobjekt auftrifft, oder/und eine Stelle des Zielobjekts, auf die bzw. den der Strahl auftrifft, vorzugsweise in Abhängigkeit von der Zeit, zu speichern.

In einer weiteren Ausführungsform der Erfindung ist die Vorrichtung, insbesondere mittels des Computers bzw. der Software, dazu vorgesehen, auf dem Zielobjekt Zielbereiche oder/und -punkte darzustellen, auf die der Strahl zu richten ist oder denen mit dem Strahl auszuweichen ist. Das Zielobjekt könnte vorgesehen sein derart, dass die Zielpunkte ihre Position darauf ändern.

Zweckmäßigerweise ist die Vorrichtung, insbesondere mittels des Computers bzw. der Software, dazu vorgesehen, abhängig von der Detektion des Orts und/oder der Dauer des Auftreffens des Strahls auf dem Zielobjekt eine Wertung zu vergeben und ggf. anzuzeigen.

In einer weiteren Ausgestaltung der Erfindung ist auf dem Computer mindestens ein Trainingsprogramm, vorzugsweise mehrere Trainingsprogramme, gespeichert, die bevorzugt mittels des Touchscreens, ausgewählt werden können. Die Trainingsprogramme können beispielsweise verschiedene Dauern und/oder Stellen, in denen der Strahl auf das Zielobjekt gerichtet werden muss bzw. auf die der Strahl zu richten ist, beinhalten. Ferner könnte vorgesehen sein, dass sich die Stellen, auf die der Strahl zu richten ist, auf dem Zielobjekt ändern und mit dem Strahl diese Stellen verfolgt werden sollen.

Der Computer kann ferner mit einer Datenbank versehen sein, auf der die Trainingsergebnisse, insbesondere die genannten Wertungen, verschiedener Trainierender gespeichert werden können.

In einer Weiterbildung der Erfindung umfasst die Vorrichtung ferner eine Messeinrichtung, mit der sich eine Kraft oder/und eine Belastung, insbesondere die Verteilung der Kraft oder/und der Belastung, die bei der Durchführung des Trainingsverfahrens durch die Fußsohle auf sie ausgeübt wird oder die an der Fußsohle anliegt, bestimmen lässt. Es versteht sich, dass der Computer auch mit der Kraft- und/oder der Belastungsverteilungsmesseinrichtung verbunden sein kann. Zweckmäßigerweise lassen sich die Informationen der Detektiereinrichtung und der Kraft- und/oder Belastungsverteilungsmesseinrichtung miteinander kombinieren, um das Trainingsergebnis noch besser beurteilen zu können.

Die Erfindung wird nachfolgend anhand von Ausführungsbeispielen und der beiliegenden Zeichnungen, die sich auf die Ausführungsbeispiele beziehen, näher erläutert. Es zeigen:
- Fig. 1: eine erfindungsgemäße Trainingsvorrichtung in Seitenansicht,
- Fig. 2: ein Detail der Trainingsvorrichtung nach Fig. 1,
- Fig. 3: die Trainingsvorrichtung nach Fig. 1 bei Durchführung des erfindungsgemäßen Trainingsverfahrens in verschiedenen Positionen,
- Fig. 4: die Trainingsvorrichtung bei Durchführung eines weiteren erfindungsgemäßen Trainingsverfahrens, und
- Fig. 5: eine Detailansicht eines weiteren Teils der erfindungsgemäßen Vorrichtung.

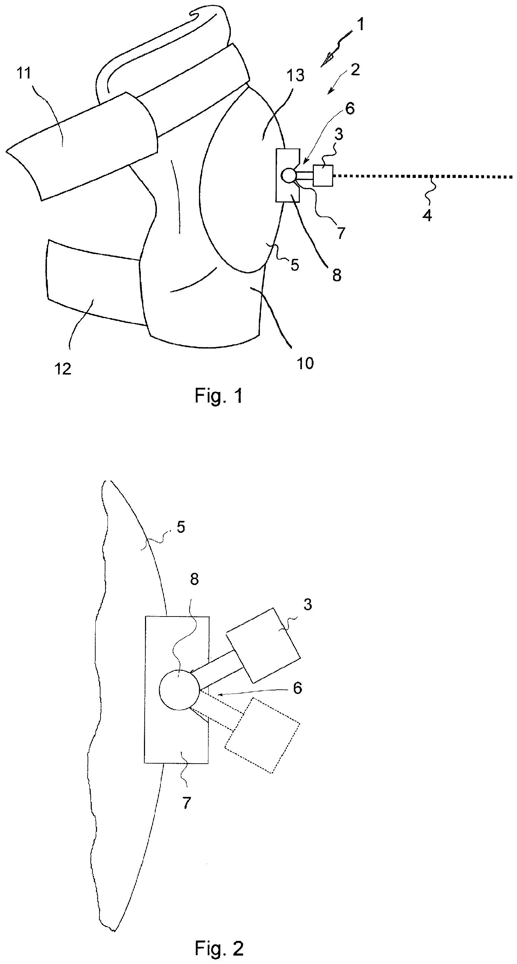

Eine in Fig. 1 gezeigte erfindungsgemäße Trainingsvorrichtung 1 umfasst eine Einrichtung 5 zu ihrer Befestigung an einem Knie des menschlichen Körpers, die durch eine Kniemanschette 5 gebildet ist. Ein Vorderteil 13 der Kniemanschette 5 lässt sich auf die Vorderseite des Knies aufsetzen und mit Bänder 11,12, die mit einem Klettverschluss versehen sein können, am Bein befestigen. Das Vorderteil 13 weist auf seiner dem Knie zugewandten Seite eine an die Form des menschlichen Knies angepasste Formung auf. Insbesondere ist auf der Innenseite der Kniemanschette eine Auswölbung gebildet, welche sich auf die Kniescheibe aufsetzen lässt.

Auf der Vorderseite der Kniemanschette ist in dem Bereich, der über der Kniescheibe anzuordnen ist, eine Strahleinheit 2 angeordnet, die einen Strahlgeber 3 und ein Gelenk 7, über welches der Strahlgeber 3 mit der Kniemanschette 5 verbunden ist, umfasst. Eine Gelenkpfanne 8 des Gelenks 7 ist unmittelbar an der Kniemanschette 5 befestigt. Der Strahlgeber 3 sitzt an dem Gelenkkopf 9, welcher verschwenkbar in der Gelenkpfanne 8 angeordnet ist.

Der Strahlgeber 3 ist durch einen Laser gebildet, der einen Strahl sichtbaren Lichts oder Infrarotlichts mit einem Durchmesser < 1 mm abgibt. Ein durch den Strahlgeber 3 abgegebener Strahl ist in Fig. 1 durch eine gestrichelte Linie dargestellt, die mit dem Bezugszeichen 4 gekennzeichnet ist. Es versteht sich, dass der Strahlgeber 3 mit einem Schalter zum Ein- und Ausschalten des Strahls versehen sein kann.

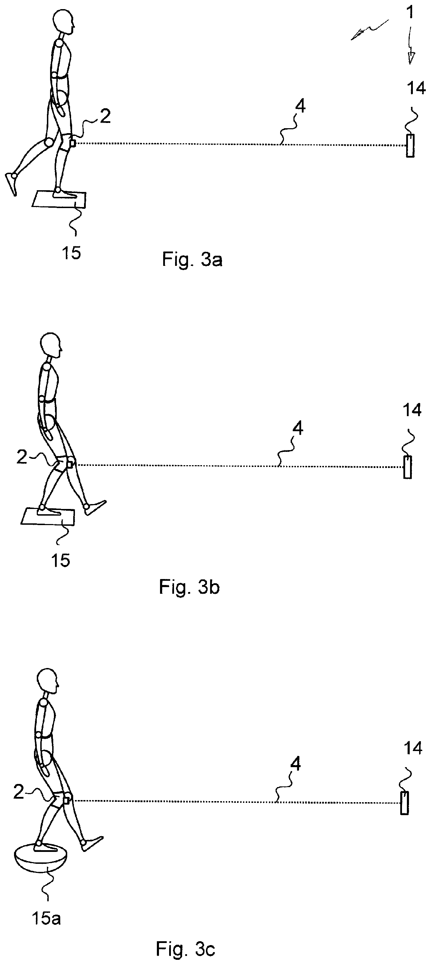

Wie insbesondere Fig. 2 zu entnehmen ist, die den Strahlgeber 3 in unterschiedlichen Positionen zeigt, kann die Ausrichtung des Strahlgebers 3 im Verhältnis zu der Kniemanschette 5 durch Verschwenkung des Strahlgebers 3 geändert werden. Dies ist von Bedeutung, um den Strahlgeber 3 so auszurichten, dass er sich bei Durchführung des Trainings- oder Testverfahrens auf ein in Fig. 3 gezeigtes Zielobjekt 14 ausrichten lässt.

Zur Durchführung eines erfindungsgemäßen Trainingsverfahrens befestigt eine Person die Trainingsvorrichtung 1 an ihrem Knie, stellt sich in der Position auf, in der das Training durchgeführt werden soll und richtet den Strahlgeber 3 durch Verschwenkung des Gelenkkopfs 7 in der Gelenkpfanne 8 so, dass der Strahl 4 auf das Zielobjekt 14 gerichtet ist. Das Zielobjekt 14 sollte dabei in ventraler Richtung vor der Person und im Abstand von mindestens 1 m, vorzugsweise mindestens 2 m, angeordnet sein. Das Training kann beispielsweise darin bestehen, auf einem festen Untergrund 15 lediglich auf dem Bein zu stehen, an dem die Kniemanschette 5 angeordnet ist, das Bein dabei zu beugen und zu versuchen, den Strahl auf das Zielobjekt 14 zu richten (vgl. Fig. 3a). Ein erweitertes Training könnte sein, dass das andere Bein in verschiedene Positionen bewegt wird, wie es beispielsweise in Fig. 3b gezeigt ist.

Wie Fig. 3c zeigt, kann das Training nicht nur auf einem festen Untergrund sondern auch auf einem instabilen Untergrund durchgeführt werden. Fig. 3c zeigt, wie die Person mit einem Bein auf einer Balancierhalbkugel 15a steht. Dadurch wird es erheblich schwieriger den Strahl 4 auf das Zielobjekt 14 zu richten und dort zu halten.

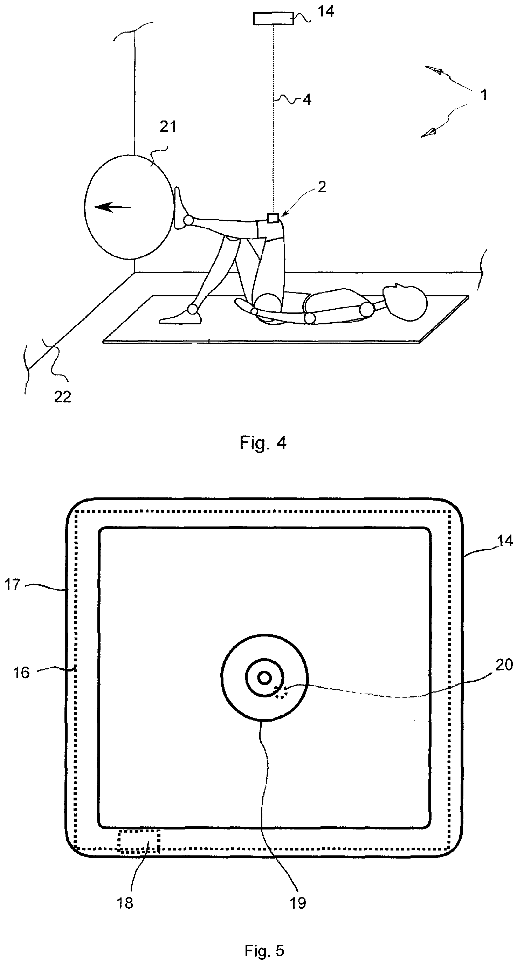

Wie in Fig. 4 dargestellt ist, kann das Trainingsverfahren auch liegend durchgeführt werden, beispielsweise wenn das Bein nicht mit dem vollen Körpergewicht belastet werden soll. Eine Möglichkeit ist, einen Ball 21 mit dem Fuß gegen eine Wand 22 zu drücken und dabei das Bein derart auszurichten, dass der Strahl 4 auf das Zielobjekt 14 gerichtet ist.

In einem weiteren, in den Zeichnungen nicht gezeigten Ausführungsbeispiel wird das Trainingsverfahren unter Benutzung einer Beinpresse durchgeführt, wobei mit einem oder mehreren Beinen unter Anwinklung des oder der Beine ein Gewicht gestemmt wird. Ein Ziel des Trainingsverfahrens kann hier sein, das Bein bzw. die Beine in einer bestimmten Ausrichtung zu halten. Insbesondere soll trainiert werden, bei Belastung des Beins bzw. der Beine unter Beugung das bzw. die Beine gerade, d.h. insbesondere parallel zur Körperlängsachse zu halten, und nicht so anzuordnen, dass das Knie bzw. die Knien medial nach innen hin oder lateral nach außen hin bewegt werden. Die Einhaltung der Ausrichtung des Beins bzw. der Beine lässt sich mittels der erfindungsgemäßen Trainingsvorrichtung 1 überprüfen, indem darauf geachtet wird, dass der Strahl beim Stemmen des Gewichts auf das Zielobjekt gerichtet wird und dorthin gerichtet bleibt. Das Zielobjekt weist dazu zweckmäßigerweise eine längliche, vorzugsweise rechteckige Form auf.

Während das Zielobjekt 14 ein beliebiger Körper sein könnte, auf dem der Strahl selbst sichtbar ist, umfasst es im vorliegenden Ausführungsbeispiel eine Sensorplatte 16 zur Erkennung des Auftreffens des Strahls 4, einen Bildschirm 17 und einen mit einer Software bespielten Mikroprozessor 18, der mit der Sensorplatte 16 und dem Bildschirm 17 verbunden ist. Das Zielobjekt 14 ist so vorgesehen, dass mittels des Mikroprozessors 18 Informationen der Sensorplatte 16 über das Auftreffen des Strahls 4 verarbeitet werden derart, dass auf dem Bildschirm 17 die Stelle angezeigt wird, an der der Strahl 4 auf das Zielobjekt 14 auftrifft. Ein solcher Anzeigepunkt ist in Fig. 5 mit der Bezugszahl 20 gekennzeichnet.

Optional kann die Trainingsvorrichtung 1 mit einem hier nicht gezeigten Lautsprecher versehen sein, der ein akustisches Signal abgibt, wenn der Strahl auf das Zielobjekt 10 auftrifft.

In der Software können verschiedene Programme vorgesehen sein, mittels derer sich auf dem Bildschirm 17 unterschiedliche Grafiken darstellen lassen. Die Grafiken können dabei verschiedene Trainingsprogramme umfassen, wobei beispielsweise ein sich bewegender Punkt 20 auf dem Bildschirm mit dem Strahl verfolgt werden muss. Ein weiteres Beispiel ist die Darstellung einer Zielscheibe 19, wobei das Ziel des Trainings ist, den Strahl 4 über eine möglichst lange Zeit im Zentrum der Zielscheibe 19 zu halten. Hierbei könnten je nach Stellen, auf die der Strahl 4 ausgerichtet wird und der jeweiligen Dauer, in der der Strahl einen bestimmten Bereich des Zielobjekts 14 trifft, automatisch bewertet werden und nach der Durchführung des Trainings automatisch eine Beurteilung abgegeben werden, die auf dem Bildschirm 17 wiedergegeben wird.

Zur besonders einfachen Bedienbarkeit ist der Bildschirm 17 als Touchscreen ausgebildet, damit sich die verschiedenen Programme einfach auswählen lassen.

In einem weiteren Ausführungsbeispiel könnte der in den Fig. 3a und b gezeigte Untergrund durch eine Platte 15 gebildet sein, die als Messeinrichtung ausgebildet ist und mittels derer sich messen lässt, wie die verschiedenen Bereiche der Sohle des Fußes, mit der die Person auf der Platte aufsteht, belastet werden. Ein Ergebnis einer solchen Messung kann mit dem Trainingsergebnis, das sich aus der Ausrichtung des Strahls 4 auf das Zielobjekt 14 ergibt, verknüpft werden, um das Training besser beurteilen zu können. Es versteht sich, dass die Ergebnisse der Messung der Belastung, beispielsweise per Funk, direkt an den Mikroprozessor 18 übertragen werden können und der Mikroprozessor 18 die Messergebnisse derjenigen der Sensorplatte 16 zur Beurteilung des Trainings zusammenführt.

## Patentansprüche

1. Vorrichtung zum Trainieren oder/und Testen des menschlichen Körpers, insbesondere zum Trainieren der Körperkoordination und/oder der Beinmuskulatur,
**gekennzeichnet durch** einen Strahlgeber (3), der zur zielgerichteten Abgabe eines Strahls (4) vorgesehen ist, und eine Einrichtung (5) zur Befestigung des Strahlgebers (3) an einem Arm oder einem Bein des menschlichen Körpers, an welcher der Strahlgeber (3) angeordnet ist.

2. Vorrichtung nach Anspruch 1,
**dadurch gekennzeichnet,**
**dass** der Strahlgeber (3) dazu eingerichtet ist, einen Strahl (4) einer elektromagnetischen Welle, insbesondere einen Lichtstrahl im Wellenlängenbereich des sichtbaren Lichts oder des Infrarotlichts, abzugeben, wobei der Strahlgeber (3) vorzugsweise ein Laser ist.

3. Vorrichtung nach Anspruch 1 oder 2,
**dadurch gekennzeichnet,**
**dass** die Befestigungseinrichtung (5) zur Anordnung an einem Gelenk des Arms oder des Beins, insbesondere des Knies, des menschlichen Körpers eingerichtet ist, wobei die Befestigungseinrichtung (5) vorzugsweise in ihrer Form an die Form angepasst ist, die der Arm oder das Bein an der Stelle, an der Befestigungseinrichtung (5) zu befestigen ist, aufweist, angepasst ist.

4. Vorrichtung nach Anspruch 3,
**dadurch gekennzeichnet,**
**dass** die Befestigungseinrichtung (5) eine Knie-, eine Handgelenk- oder eine Ellbogenmanschette oder eine Knie-, eine Handgelenk- oder eine Ellbogenorthese umfasst.

5. Vorrichtung nach Anspruch 4,
**dadurch gekennzeichnet,**
**dass** der Strahlgeber (3) an der Kniemanschette oder der Knieorthese an einer Stelle befestigt ist, die zur Anordnung an oder neben einer Kniescheibe, insbesondere auf der Kniescheibe, vorgesehen ist.

6. Vorrichtung nach einem der Ansprüche 1 bis 5,
**dadurch gekennzeichnet,**
**dass** der Strahlgeber (3) eine Einrichtung (6) zur Verstellung seiner Position im Verhältnis zu der Befestigungseinrichtung (5) aufweist, um die Richtung, in welcher der Strahlgeber (3) den Strahl (4) abgibt, einzustellen.

7. Vorrichtung nach einem der Ansprüche 1 bis 6,
**dadurch gekennzeichnet,**
**dass** die Verstelleinrichtung (6) ein Gelenk (7), vorzugsweise Drehgelenk oder ein Kugelgelenk, umfasst.

8. Vorrichtung nach einem der Ansprüche 1 bis 7,
**dadurch gekennzeichnet,**
**dass** die Vorrichtung ein Zielobjekt (14), auf den der Strahl (4) zu richten ist, umfasst und das Zielobjekt (14) vorzugsweise eine Detektiereinrichtung (16) aufweist, die dazu eingerichtet ist, ein Auftreffen des Strahls (4) zu erkennen.

9. Vorrichtung nach einem der Ansprüche 1 bis 8,
**dadurch gekennzeichnet,**
**dass** die Vorrichtung eine Meldeeinrichtung umfasst, die dazu eingerichtet ist, zu melden, wenn der Strahl auf das Zielobjekt auftrifft, wobei die Meldeeinrichtung vorzugsweise zur Abgabe eines optischen und/oder eines akustischen Signals vorgesehen ist.

10. Vorrichtung nach einem der Ansprüche 1 bis 9,
**gekennzeichnet durch** einen Computer, der mit dem Zielobjekt, der Detektiereinrichtung und/oder der Meldeeinrichtung verbunden ist, wobei auf dem Computer vorzugsweise eine Software gespeichert ist, wobei der Computer oder/und die Software bevorzugt dazu eingerichtet ist, die Meldeeinrichtung anhand der mittels der Detektiereinrichtung ermittelten Informationen zur Abgabe des optischen und des akustischen Signals zu veranlassen.

11. Vorrichtung nach einem der Ansprüche 1 bis 10,
**dadurch gekennzeichnet,**
**dass** die Vorrichtung, insbesondere mittels des Computers und/oder der Software, dazu vorgesehen ist, eine Dauer, innerhalb derer der Strahl auf das Zielobjekt auftrifft, oder/und eine Stelle des Zielobjekts, auf die bzw. den der Strahl auftrifft, vorzugsweise in Abhängigkeit von der Zeit, zu speichern.

12. Vorrichtung nach einem der Ansprüche 1 bis 11,
**dadurch gekennzeichnet,**
**dass** auf dem Computer mindestens ein Trainingsprogramm gespeichert ist, die bevorzugt verschiedene Dauern und/oder Stellen, in denen der Strahl auf das Zielobjekt gerichtet werden muss oder auf die der Strahl zu richten ist, umfassen.

13. Vorrichtung nach einem der Ansprüche 1 bis 12,
**gekennzeichnet durch** eine Messeinrichtung (15), mit der sich eine Belastung, vorzugsweise eine Verteilung der Belastung, die bei der Durchführung des Trainingsverfahrens **durch** die Fußsohle auf sie ausgeübt wird, bestimmen lässt.

14. Verfahren zum Trainieren oder/und Testen des menschlichen Körpers, insbesondere zum Trainieren der Körperkoordination und/oder der Beinmuskulatur,
**dadurch kennzeichnet,**
**dass** ein Strahlgeber (3) an einem Arm oder einem Bein des menschlichen Körpers befestigt wird und ein von dem Strahlgeber (3) ausgegebener Strahl (4) auf ein Zielobjekt (14) gerichtet wird.

15. Verfahren nach Anspruch 14,
**dadurch gekennzeichnet,**
**dass** detektiert wird, wenn der Strahl (4) auf das Zielobjekt (14) auftrifft.
